(19) Europäisches Patentamt
European Patent Office
Office européen des brevets

(11) EP 1 212 271 B1

(12) **FASCICULE DE BREVET EUROPEEN**

(45) Date de publication et mention de la délivrance du brevet:
**14.01.2004 Bulletin 2004/03**

(21) Numéro de dépôt: **00962635.9**

(22) Date de dépôt: **14.09.2000**

(51) Int Cl.7: **C07C 43/174**, C09K 11/06, H01L 51/30, C08G 61/10

(86) Numéro de dépôt international:
**PCT/FR2000/002538**

(87) Numéro de publication internationale:
**WO 2001/019765 (22.03.2001 Gazette 2001/12)**

(54) **MONOMERES, POLYMERES INCORPORANT LESDITS MONOMERES ET LEUR UTILISATION AU SEIN DE DISPOSITIFS ORGANIQUES ELECTROLUMINESCENTS**

MONOMERE UND SIE ENTHALTENDE POLYMERE SOWIE IHRE VERWENDUNG IN ORGANISCHEN ELEKTROLUMINESZENTEN ELEMENTEN

MONOMERS, POLYMERS INCORPORATING SAID MONOMERS AND THEIR USE IN ORGANIC ELECTROLUMINESCENT DEVICES

(84) Etats contractants désignés:
**AT BE CH CY DE DK ES FI FR GB GR IE IT LI LU MC NL PT SE**

(30) Priorité: **15.09.1999 FR 9911702**

(43) Date de publication de la demande:
**12.06.2002 Bulletin 2002/24**

(73) Titulaire: **UNIVERSITE JOSEPH FOURIER - Grenoble 1**
**38400 St. Martin d'Hères (FR)**

(72) Inventeurs:
- **STEPHAN, Olivier**
  **La Haute Frette 38660 LE TOUVET (FR)**
- **ARMAND, Michel**
  **Montréal, Québec H2T 2N2 (CA)**
- **VIAL, Jean-Claude**
  **38000 Grenoble (FR)**

(74) Mandataire: **Vuillermoz, Bruno**
**Cabinet Laurent & Charras**
**B.P. 32**
**20, rue Louis Chirpaz**
**69131 Ecully Cédex (FR)**

(56) Documents cités:
**WO-A-97/33323**
**US-A- 4 226 967**
**US-A- 5 708 130**

- **DO-HOON HWANG ET AL: "New luminescent polymers for LEDS and LECS" MACROMOLECULAR SYMPOSIA, vol. 125, 1997, pages 111-120, XP000907043**
- **BENG SIM CHUAH ET AL: "New luminescent polymers for LEDS" SYNTHETIC METALS, vol. 91, no. 1-3, décembre 1997 (1997-12), pages 279-282, XP000910726**
- **R. O. GARAY ET AL: "Synthesis and characterization of poly[2,5-bis(triethoxy)-1,4-phenylene vinylene]" JOURNAL OF POLYMER SCIENCE, PART A: POLYMER CHEMISTRY, vol. 33, no. 3, février 1995 (1995-02), pages 525-531, XP002138825**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne un nouveau monomère, de même que le polymère (homopolymère ou copolymère) incorporant ledit monomère, ainsi que leur utilisation au sein de dispositifs organiques électroluminescents.

Parmi les dispositifs organiques électroluminescents, on distingue les diodes électroluminescentes encore connues sous le terme de « LED » signifiant Light-Emitting Diode, des cellules électrochimiques électroluminescentes encore désignées « LEC », signifiant Light Emitting Electrochemical Cell.

Les LEDs et les LECs sont bien connues et correspondent à des émetteurs constitués d'une couche mince de polymère définissant une zone dite « active », laquelle est prise en sandwich entre deux électrodes, respectivement une cathode et une anode, dont une au moins est transparente ou semi-transparente facilitant ainsi l'observation de l'émission lumineuse. La structure en film mince de ces émetteurs est particulièrement avantageuse pour l'éclairage diffus et les écrans plats.

En pratique, l'anode semi-transparente est réalisée en ITO signifiant Indium Tin Oxide, résultant du dopage de $In_2O_3$ par $SnO_2$, ou en oxyde d'étain dopé par l'antimoine ou le fluor ($SnO_2$:Sb ou $SnO_2$:F), tandis que la cathode est réalisée en aluminium.

Les polymères mis en oeuvre au sein de la zone active sont des polymères dits « conjugués », c'est-à-dire des polymères dont les monomères constitutifs présentent une alternance de liaisons simples, dites liaison $\sigma$ et doubles, dites liaisons $\pi$, conduisant à un système d'électrons $\pi$ fortement délocalisés le long de la chaîne carbonée.

De tels composés sont principalement dérivés du polyacétylène ou encore obtenus par enchaînement de noyaux aromatiques tels que le benzène, le naphtalène, le pyrrole, le thiophène, la pyridine, la quinoline, l'anthracène, le carbazole et le fluorène.

Dans certains cas, pour permettre de diminuer les contraintes stériques et ainsi maintenir les entités aromatiques dans le même plan, les noyaux précités sont couplés par le biais d'une liaison vinylène -(CH=CH)-.

D'un point de vue électrochimique, les polymères conjugués peuvent, moyennant un réarrangement structurel de l'alternance de leurs liaisons, être oxydés ou réduits. Plus précisément, l'oxydation, c'est-à-dire la perte d'un électron peut être interprétée comme l'apport d'un trou dans la bande de valence (HOMO), soit un dopage "p" du matériau. De même, la réduction consistant en l'ajout d'un électron peut être interprétée comme étant l'apport d'un électron dans la bande de conduction (LUMO), soit un dopage "n". Dès lors, la différence entre le potentiel d'oxydation et de réduction du polymère conjugué peut être analysée comme correspondant au gap du polymère en tant que matériau semi-conducteur.

Du fait de ses propriétés semi-conductrices, ce type de polymères peut être mis en oeuvre dans les dispositifs organiques électroluminescents décrits ci-avant, le phénomène d'électroluminescence résultant de la recombinaison radiative d'un électron issu de la bande de conduction et d'un trou issu de la bande de valence, les porteurs (trous, électrons) étant injectés dans la couche active lors de l'application d'un potentiel entre les deux électrodes.

Les LEDs et les LECs se distinguent de par la composition de leur zone active.

Ainsi, alors que la zone active des LEDs est exclusivement constituée d'un polymère ne contenant pas d'additif de conductivité, obtenu sous forme de film à partir d'une solution dans un solvant ou par évaporation sous vide, la couche des LECs comprend en outre un sel et un solvant de ce sel mélangé au polymère conjugué ou greffé sur celui-ci.

Cette différence de composition a pour effet de conférer à ces deux dispositifs des tensions de fonctionnement différentes. Ainsi, s'agissant des LEDs, il est nécessaire d'appliquer une tension de fonctionnement élevée supérieure à 10 volts, alors qu'une tension de fonctionnement comprise entre 3 et 4 volts est suffisante pour les LECs.

La tension élevée nécessaire au fonctionnement des LEDs est due à la très faible conductivité des polymères, utilisés à l'état non dopé et au caractère non-ohmique des contacts entre polymère et électrodes, ce caractère non-ohmique résultant de l'existence de barrières de potentiels.

L'adjonction d'un sel dans la couche mince des LECs permet de diminuer la hauteur de cette barrière de potentiel d'après le phénomène suivant.

Lorsqu'un faible potentiel est appliqué entre l'anode et la cathode, les ions issus du sel dissocié migrent vers les électrodes concernées pour former deux fines couches chargées à l'interface du milieu actif et des électrodes, couches qui favoriseront l'injection des électrons et des trous en rendant le polymère conducteur aux interfaces polymère/électrodes. Lorsque le potentiel appliqué devient supérieur à la tension seuil, les électrons et les trous sont injectés respectivement aux interfaces anode/couche active et cathode/couche active, initiant ainsi la formation d'une jonction p-n. Lorsqu'on augmente à nouveau la tension, les électrons et les trous supplémentaires injectés migrent sous l'effet de cet excès de potentiel vers la cathode et l'anode. La recombinaison radiative de ces porteurs au sein de la zone de charge d'espace est à l'origine du phénomène d'électroluminescence.

Lorsque le dopage est suffisant, le contact entre les électrodes et le polymère est de nature ohmique, de sorte que les tensions de fonctionnement sont considérablement abaissées et proches de la valeur théorique, soit la différence énergétique entre la bande de valence (HOMO) et la bande de conduction (LUMO) du polymère.

Toutefois, la réalisation de la couche mince ou zone active des LECs n'est pas simple dans la mesure où

ladite couche est susceptible de contenir un matériau polymère hydrophobe pratiquement non polaire et une espèce ionique, qui n'est dissociée appréciablement qu'en présence d'un solvant polaire.

Pour permettre le mélange du polymère hydrophobe avec le sel hydrophile, on a proposé de solvater cations et anions résultant de la dissolution du sel.

Pour ce faire, une première solution consiste à solvater le sel, tel que le sel de lithium, par exemple de l'acide trifluorométhane sulfonique ($LiCF_3SO_3$) au moyen d'un polymère solvatant du type poly(oxyde d'éthylène). Cependant, les deux polymères sont immiscibles, et d'autant moins compatibles en présence de sel, de sorte que le mélange obtenu se présente sous forme hétérogène.

Une autre méthode décrite dans le document WO 97/33323 consiste à greffer des segments solvatants de type oligo (oxyde d'éthylène) sur la trame d'un polymère conjugué du type fluorène. Si ce type de composé permet de résoudre le problème de l'inhomogénéité du mélange polymère (microséparation de phase), la conjugaison existant entre les différentes unités monomères est cependant susceptible d'être modifiée.

En effet, les segments solvatants de type oligo (oxyde d'éthylène) induisent un désordre local, qui, nécessaire à la conduction ionique, se traduit par une perte de coplanéarité des unités permettant la conjugaison $\pi$. De plus, le désordre local est encore accentué par l'adjonction d'un composé ionique, l'arrangement des chaînes latérales se faisant en priorité pour assurer la solvatation des cations $Li^+$. De plus, l'énergie mise en oeuvre par ce processus (supérieure à 60 KJ) est nettement supérieure au gain d'énergie obtenu grâce à l'extension de la conjugaison $\pi$ (supérieure à 10 KJ).

Ainsi, par exemple, les polythiophènes substitués en position 3 par des groupements oligo (oxyde d'éthylène) de masse proche de 200 changent leur spectre d'absorption optique en passant du violet au jaune en présence de cations alcalins, ce qui prouve la perte de la conjugaison $\pi$.

Parallèlement, la diminution de la conjugaison est préjudiciable à la conductivité électronique, laquelle nécessite un ordre local, ainsi qu'à la fluorescence et la luminescence.

Par ailleurs, la diminution de la conjugaison rend plus difficiles les dopages "n" et "p", qui se produisent à des potentiels plus cathodiques et plus anodiques que les polymères non substitués par des segments oligo (oxyde d'éthylène). Dès lors, les matériaux dopés sont moins stables, en particulier vis-à-vis des agents tels que l'eau ou l'oxygène de l'air.

Enfin, les groupements $OCH_2CH_2$, correspondant à la fixation directe des segments oligo (oxyde d'éthylène) sur le polymère, ont un pouvoir attracteur dû à l'électronégativité de l'oxygène contribuant ainsi à rendre plus difficile le dopage "p".

Le problème que se propose de résoudre l'invention est de fournir un monomère présentant un noyau aromatique muni de segments solvatants, qui puisse être polymérisé et qui ne présente pas les inconvénients ci-avant décrits.

Pour ce faire, l'invention propose un monomère de formule générale

$$A\text{-}(Q\text{-}S)_p$$

selon laquelle :

A = noyau aromatique ou hétéroaromatique,
Q = radical divalent carboné ou silicié,
S = chaîne aliphatique comprenant au moins un hétéroatome polaire,

$$1 \leq p \leq 6$$

En d'autres termes, l'invention consiste à avoir séparé le noyau aromatique A du monomère du segment solvatant S par l'intermédiaire d'un lien chimique flexible Q permettant ainsi de séparer dans l'espace les fonctions solvatantes des fonctions liées à la conjugaison. Cette solution permet de résoudre les problèmes précédemment évoqués. En effet, le système conjugué peut adopter une conformation plane, donc à l'optimal de ses propriétés de conjugaison, dopage, conductivité, luminescence. Les segments solvatants peuvent, indépendamment de toute contrainte sur le système conjugué, se conformer pour fournir une solvatation optimale des cations, et donc la meilleure conduction ionique. Le bras espaceur Q n'étant pas attracteur d'électron influe très peu sur le potentiel de dopage, permettant ici aussi de garder une stabilité du polymère dopé.

Selon une première caractéristique de l'invention, A est choisi dans le groupe comprenant les noyaux phényle, naphtalène, pyrrole, thiophène, benzothiophène, pyridine, quinoline, carbazole, anthracène et fluorène.

Pour améliorer la conjugaison du système d'électrons $\pi$ délocalisés, le noyau aromatique ou hétéromatique

A peut être engagé dans différents types de liaisons.

Cette liaison peut par exemple se faire sous forme d'une chaîne comprenant 2n atomes, tels que C ou N et "n" doubles liaisons conjuguées avec S.

Parmi ces liaisons, on met avantageusement en oeuvre des liaisons du type vinylène $(CR=CH)_n$ avec n compris entre 1 et 5 et R = H, alkyle, aryle comprenant 1 à 12 atomes de carbone. Dans ce cas, A est choisi dans le groupe comprenant le vinylène-phényle, vinylène-naphtalène, vinylène-pyrrole, vinylène-thiophène, vinylène-benzothiophène, vinylène-pyridine, vinylène-quinoline, vinylène-carbazole, vinylène-fluorène et vinylène-anthracène.

La liaison peut également être une liaison azométhine $(CR=N)_n$ ou encore azo $(N=N)_n$.

Selon une autre caractéristique de l'invention, le monomère présente comme déjà dit des segments solvatants S sous forme d'une chaîne aliphatique comprenant au moins un hétéroatome polaire tel O, N, S ou P possédant des doublets électroniques libres susceptibles d'interagir avec les cations issus de la dissociation du sel.

Selon un premier mode de réalisation, la chaîne aliphatique S est un polyéther de formule générale :

$$-[OCH_2CH(R)]_rOR'\text{- ou } -[OCH(R)CH_2]_rOR'$$

avec r compris entre 1 et 50

R = alkyle comprenant de 1 à 6 atomes de carbone
R' = alkyle, alkényle, aryle, aryle alkyle, alkényle aryle comprenant de 1 à 30 atomes de carbone

Dans une seconde forme de réalisation, S est une polyamine correspondant à la formule générale :

$$-[N(R'')CH_2CH(R)]_rN(R'')R'\text{- ou } -[OCH(R)CH_2]_rN(R'')R'$$

où :

r compris entre 1 et 50
R et R" = H, alkyle comprenant de 1 à 6 atomes de carbone
R' = alkyle, alkényle, aryle, aryl alkyle, alkényl aryle comprenant de 1 à 30 atomes de carbone

Dans une forme de réalisation avantageuse, S est un polyéther correspondant aux formules générales ci-dessus et pour lequel R = R" = H ou $CH_3$ et R' = méthyle, éthyle, butyle, hexyle, octyle, dodécyle, tétradécyle, hexadécyle benzyle.

Comme déjà dit, pour obtenir une meilleure conductivité du monomère, les segments solvatants sont séparés du noyau aromatique par l'intermédiaire de liaisons flexibles.

Dans une première forme de réalisation, le radical divalent Q constituant la liaison chimique flexible correspond à la formule générale $(CR^1R^2)_n$ avec $R^1$, $R^2$ = H, alkyle, alkényle comprenant entre 1 et 4 atomes de carbone et n compris entre 4 et 24.

Avantageusement, la liaison flexible Q présente la formule générale $(CH_2)_n$ avec n compris entre 4 et 24 atomes de carbone.

Dans une autre forme de réalisation, le radical divalent Q constituant la liaison chimique flexible correspond à la formule générale $[(O\text{-}Si(R_1R_2)]_n$ avec $R_1$,$R_2$ = H, alkyl, alkényl comprenant entre 1 et 4 atomes de carbone et n compris entre 3 et 24

Par ailleurs et selon une autre caractéristique, le monomère de l'invention présente des fonctions aptes à permettre sa polymérisation ultérieure tels que par exemple des fonctions halogénées, amines ou aldéhydes permettant une polycondensation.

Dans des formes de réalisations avantageuses, le monomère de l'invention est le 2,7-dibromo-9,9-bis (7,10,13,16 tétraoxaheptadécyl)fluorène ou le 2,7-dibromo-9,9-bis (5,8,11-trioxadodécyl)fluorène.

Dès lors, l'invention concerne également un polymère de type homopolymère ou copolymère incorporant le monomère de l'invention. Ce polymère peut être aléatoire, à caractère aléatoire, alterné ou à caractère alterné, à bloc ou à caractère bloc.

Les méthodes permettant le couplage et donc la polymérisation des monomères de l'invention, soit directement, soit par l'intermédiaire des liaisons énumérées précédemment (notamment du type vinylique), sont parfaitement connues de l'homme du métier.

On peut citer par exemple le couplage de monomères portant deux groupements halogènes ou pseudo-halogènes en présence d'un agent réducteur et d'un catalyseur, par exemple dérivés de la 2-2' bipyridine ou encore,

dérivés du nickel ou du palladium complexé par des phosphines tertiaires.

Les monomères présentant des liaisons vinylènes sont obtenus par élimination d'un groupement sulfonium, obtenu à partir du dérivé halogéné en milieu basique selon la réaction suivante :

$$\sim\sim\sim \text{A-CH}_2\text{X} + \text{S(R}^5)_2 \rightarrow [\sim\sim\sim \text{A-CH}_2\text{S(R}^5)_2]^+ + \text{X}^-$$

$$[\sim\sim\sim \text{A-CH}_2\text{S(R}^5)_2]^+ + \text{X}^- + {}^-\text{X}^+[\text{S(R}^5)_2\text{-CH}_2\text{-A} \sim\sim\sim]^+ + 2\text{OH}^- \rightarrow$$

$$\sim\sim\sim \text{A-CH=CH-A} \sim\sim\sim + 2\text{H}_2\text{O} + 2\text{S(R}^5)_2 + 2\text{X}^-$$

où :

- X représente un halogène ou un pseudo halogène, Cl, Br, I, SCN, $NO_2$, $CF_3SO_2$, $CF_3SO_3$.
- $R^5$ représente un groupement alkyle de 1 à 4 atomes de carbone, ou bien deux groupements $R^5$ sont réunis avec le soufre pour former un cycle, en particulier dans le cas où $S(R^5)_2$ est le tétrahydrothiophène.
- $\sim\sim\sim$ représente la chaîne macromoléculaire en croissance.

Il est également possible d'effectuer une polymérisation par élimination duplicative :

$$\sim\sim\sim \text{A-CH}_2\text{X} + \text{X-CH}_2\text{A} \sim\sim\sim + 2\text{R}^t\text{OM} \longrightarrow \sim\sim\sim \text{A-CH=CH-A} \sim\sim\sim + 2\text{XM} + 2\text{R}^t\text{OH}$$

où :

- X ayant les significations ci-dessus
- $R^t$ étant un radical organique carboné tertiaire
- M est un métal alcalin ou alcalino-terreux

De préférence, $R^t$ est le groupement tertiobutyle ou tertioamyle.

L'invention se rapporte également à un dispositif organique électroluminescent constitué d'une couche mince comprenant le polymère de l'invention, ladite couche étant prise en sandwich entre deux électrodes.

Bien entendu, le polymère électroluminescent peut être intégré dans la zone active aussi bien de LEDs que de LECs.

Cependant, dans la suite de la description, le polymère de l'invention est plus particulièrement utilisé dans les LECs. Dans ce type de dispositif, la couche mince comprend, en plus du polymère, un sel.

Dès lors et comme déjà dit, la création d'une jonction ohmique aux interfaces avec les électrodes, et l'absence de charges d'espace résultent de la formation de couches dopées n et p, respectivement à l'électrode négative et l'électrode positive.

Or, le polymère de l'invention, grâce à la séparation des segments solvatants et des noyaux aromatiques, permet d'obtenir une conjugaison optimale du système $\pi$, tout en respectant les caractéristiques de solvatation apportées par les groupements S.

En pratique, les cations issus de la dissociation du sel compris dans la couche mince constituant la zone active de la LEC sont choisis dans le groupe comprenant les cations métalliques et des cations de type omium. Parmi les cations métalliques, on choisit les cations dérivés des terres rares et des métaux de transition, avantageusement les cations dérivés de métaux alcalins et des métaux alcalino-terreux.

Par ailleurs, par l'expression « cations de type omium », on désigne les cations ammonium ($NRi_{4+}$), phosphonium ($PRiH_{4+}$), amidinium ($RC(NRiH_2)_{2+}$) ou guanidinium ($C(NRiH_2)_{3+}$) avec R,Ri = H, alkyle, aryle comprenant 1 à 18 atomes de carbone.

Bien entendu, la solubilité des sels correspondant aux cations précités, ainsi que la dissociation et la conductivité, dépendent fortement de l'anion correspondant. On préférera en particulier les anions de faible basicité et correspondant aux acides forts. Parmi ceux-ci, on choisira en particulier $ClO_4$-, $BF_4$-,$PF_6$-, $AsF_6$-, $SbF_6$-, $TeOF_5$-,$RSO_{3-}$, $(RSO_2)_2N^-$, $(RSO_2)_2CY^-$, où R est un radical principalement perfluoré comprenant de 1 à 8 atomes de carbone, Y = H, CN, $RSO_2$.

Selon une autre caractéristique, le matériau de l'électrode transparente est choisi dans le groupe comprenant l'oxyde de zinc dopé par du zinc métallique, le gallium, le zirconium, l'oxyde d'étain dopé par du fluor ou de l'antimoine,

l'oxyde d'indium dopé par de l'étain, l'antimoine et le fluor.

De façon connue, ces oxydes peuvent être déposés soit sur des couches de verre ou d'un polymère, soit directement sur le matériau électroluminescent, c'est-à-dire le polymère de l'invention par des techniques telles que la pulvérisation cathodique.

Par ailleurs, il est également avantageux de choisir au moins une électrode ayant un pouvoir réflecteur élevé, constitué de préférence d'un métal ou d'un alliage.

On utilise en particulier un métal choisi dans le groupe comprenant l'aluminium, l'or, l'argent, l'étain, le plomb, le béryllium, le magnésium, le calcium, le baryum, ainsi que leurs alliages.

Pour augmenter l'adhésion ou immobiliser les molécules actives électrochimiquement ou optiquement, les électrodes peuvent être avantageusement traitées en surface par des procédés d'ensimage, notamment avec des organosilanes.

De même, pour modifier les propriétés d'injection des porteurs (électrons, trous), les électrodes sont recouvertes d'une couche d'un matériau organique ou inorganique.

Dans une forme de réalisation avantageuse, le composé constituant la couche facilitant l'injection de porteurs est choisi dans le groupe comprenant les polyquinolines, les polycarbazoles, les polymères possédant des noyaux aromatiques liés par des groupements cyanovinylènes C(CN)=CH-, les polyanylines, les polythiophènes et notamment ceux possédant des fonctions substituant éther phényle dans la position 3 et/ou éther dans la position 4, les fluorures, tel que LiF, $MgF_2$, les oxydes tel que MnO, les nitrures tels que AlN ou $Si_3N_4$.

Les dérivés des monomères de l'invention peuvent être additionnés de différents adjuvants. Ce peut être des particules solides telles que des nanoparticules de céramique, des oxydes comme la silice, l'alumine $Al_2O_3$, $LiAlO_2$, ZnO, $SnO_2$, les nitrures tels que AlN. Ce peut être également des polymères sous forme de latex ou de fibres, telles que le polystyrène, le polyvinylpyrrolidinone, la polyvinylpyridine, le polypropylène, le polyéthylène. On peut également envisager l'addition de polyoxydes d'éthylène et de ses copolymères, éventuellement en présence d'agents dispersants, de même que l'addition de plastifiants tels que esters de polyacides organiques, éthers et esters d'oligo (oxyde d'éthylène).

L'invention et les avantages qui en découlent ressortiront mieux des exemples de réalisation suivants.

**Exemple 1 : Synthèse du copolymère poly[(9,9-bis(7,10,13,16-tétraoxahéptadécyle)fluorène-co-9,9-(dihéxyl) fluorène)]**

**1/ Synthèse du groupement fonctionnel 1-bromo-7,10,13,16-tétraoxahéptadécyle**

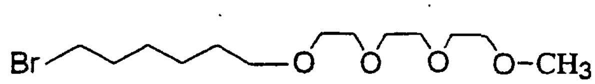

4,92 g (30 mmoles) de triéthylèneglycol monométhyléther sont diluées dans 80 ml de tétrahydrofurane anhydre. On ajoute ensuite 1 équivalent (0,72 g) de sodium métallique (Na). Le mélange est ensuite agité à température ambiante pendant 12 heures. L'alcoolate ainsi formé est transféré dans une ampoule à brome et ajouté goutte à goutte, à température ambiante, dans un mélange de 50 ml de tétrahydrofurane et de dibromohéxane en large excès (36,6 g soit 5 équivalents). Le mélange réactionnel est ensuite laissé sous agitation 12 heures supplémentaires. La solution organique ainsi obtenue est neutralisée avec 10 ml d'eau. Le 1-bromo-7,10,13,16-tétraoxahéptadécyle est ensuite extrait à l'éther. On lave ensuite abondamment cette phase organique avec une solution aqueuse. La phase organique est ensuite séchée sur $MgSO_4$ puis évaporée-sous pression réduite. On obtient une huile jaunâtre contenant principalement un mélange de 1-bromo-7,10,13,16-tétraoxahéptadécyle et de dibromohéxane en excès.

La purification est réalisée par chromatographie sur colonne de silice en deux étapes : une première élution réalisée avec du pentane permet d'éliminer le dibromohéxane du mélange. Le 1-bromo-7,10,13,16-tétraoxahéptadécyle est ensuite relargué en utilisant comme éluant un mélange pentane/acétone (50/50). Après évaporation on obtient une huile de couleur jaune.

**2/ Synthèse du monomère 2,7-dibromo-9,9-bis(7,10,13,16-tétraoxahéptadécyl)fluorène.**

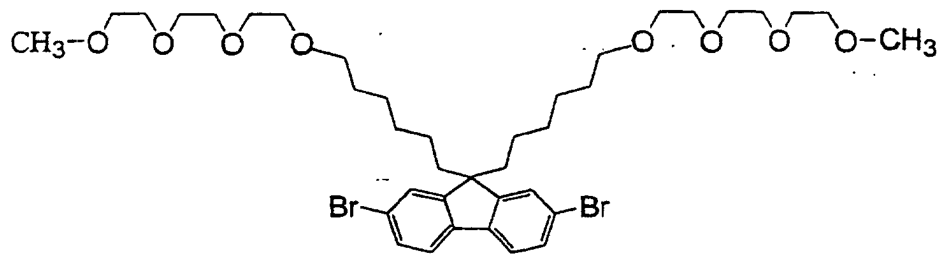

On dissout 5 mmoles (1,62g) de 2,7-dibromofluorène dans 30 ml de N,N-diméthylformamide anhydre. 10 mmoles d'hydrure de sodium (NaH), 240 mg. sont alors rajoutées progressivement à cette solution. Après agitation du mélange, à température ambiante, pendant six heures, 10 mmoles (2,27 g) de 1-bromo-7,10,13,16-tétraoxahéptadécyle sont ajoutées goutte à goutte. Le mélange réactionnel est ensuite agité à température ambiante pendant une période de 12 heures. Le mélange réactionnel est ensuite neutralisé avec 10 ml d'eau. Le monomère est extrait à l'éther. On lave ensuite cette phase organique avec une solution aqueuse. La phase organique est ensuite séchée sur $MgSO_4$ puis évaporée sous pression réduite. On obtient une huile de couleur jaune. La purification est réalisée par chromatographie sur colonne de silice en utilisant du pentane ou de l'héxane comme éluant.

**3/ Synthèse du poly[9,9-bis(7,10,13,16-tétraoxahéptadécyle)fluorène-2,7-diyl]**

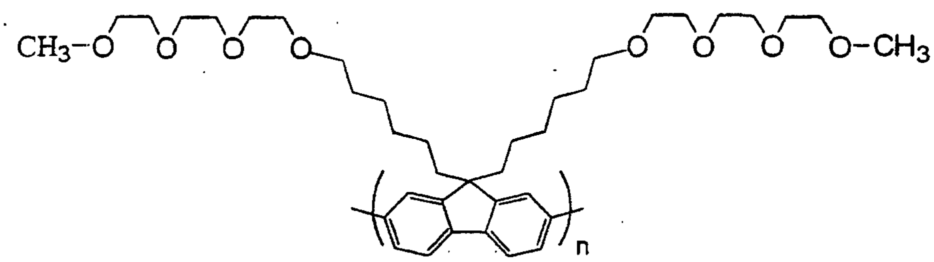

2,5 mmoles (2,04 g) de 2,7-dibromo-9,9-bis(7,10,13,16-tétraoxahéptadécyle)fluorène, 0,25 mmoles (65,5 mg.) de triphényle phosphine, 0,125 (19,52 mg.) mmoles de 2,2'-dipyridyle, 0,125 mmoles (16,20 mg.) de chlorure de nickel $NiCl_2$, 7,75 mmol (506 mg.)de poudre de zinc (99,998 % - 100 mesh) sont introduites dans un ballon de 20 ml. 3 ml de N,N-diméthylacétamide anhydre sont ensuite ajoutés. Le mélange tout d'abord agité pendant 30 minutes à température ambiante et puis porté à une température de 80 °C. L'agitation est maintenue pendant 3 jours à l'abri de la lumière. Après refroidissement la solution obtenue est reprise par 5 ml d'un mélange méthanol/HCl concentré puis évaporée à sec. Le polymère est ensuite redissout dans de l'héxane. Après évaporation on récupère une huile jaune : le polymère est fondu à température ambiante

4/ Synthèse du copolymère (proportion initiale des monomères 1 : 4 ) poly[(9,9-bis(7,10,13,16-tétraoxahéptadécyle) fluorène-co-9,9-(dihéxyl)fluorène)]

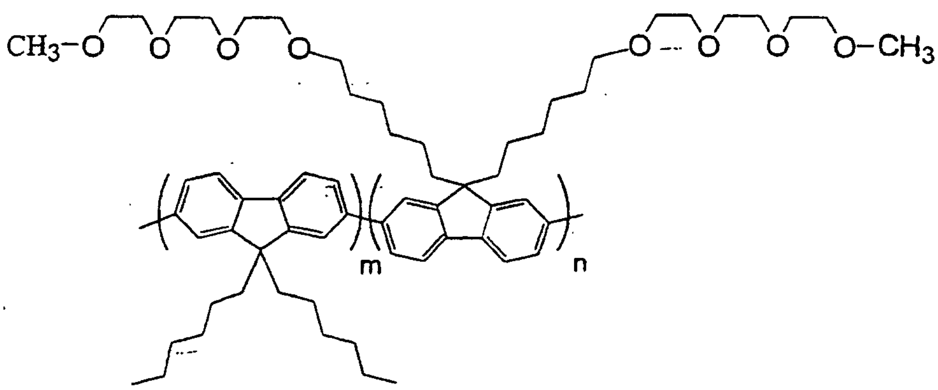

0,5 mmol (312 mg.) de 2,7-dibromo-9,9-bis(7,10,13,16-tétraoxahéptadécyle)fluorène, 2,0 mmol (661 mg.)de 2,7-dibromo-9,9-(dihéxyle)fluorène, 0,25 mmol de triphényle phosphine ; 0,125 mmol de 2,2'-dipyridyle, 0,125 mmol de chlorure de nickel $NiCl_2$, 7,75 mmol de poudre de zinc (99,998 % - 100 mesh) sont introduites dans un ballon de 20 ml. 3 ml de N,N-dimethylacétamide anhydre sont ensuite ajoutés. Le mélange tout d'abord agité pendant 30 minutes à température ambiante et puis porté à une température de 80 °C. L'agitation est maintenue pendant 3 jours à l'abri de la lumière. Après refroidissement la solution obtenue est versée dans 100 ml d'un mélange méthanol/HCl concentré (80/20). Le polymère qui précipite alors est récupéré par filtration puis séché sous vide primaire. Une purification plus poussée peut-être effectuée en renouvelant cette opération plusieurs fois. Dans ces conditions le solide est redissout dans 5 ml de N,N-diméthylacétamide. Le copolymère obtenu se présente sous forme d'un solide jaune clair.

**Exemple 2 : Synthèse du monomère 2,7-dibromo-9,9-bis(5,8,11,-trioxadodécyl)fluorène**

**1/ Synthèse du 1-bromo-5,8,11,-trioxadodécane :**

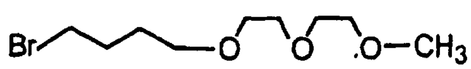

Ce composé est préparé d'une manière similaire au paragraphe 1/ de l'exemple 1) par réaction du sel de sodium du diéthylèneglycol monométhyléther sur un excès de 1,4- dibromobutane. Après purification sur colonne, on obtient une huile de couleur jaune.

**2/ Synthèse du monomère 2,7-dibromo-9,9-bis(5,8,11,-trioxadodécyl)fluorène :**

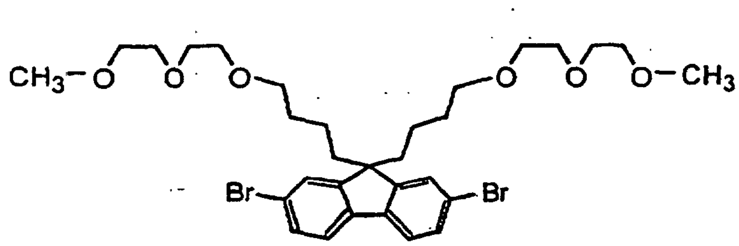

2 équivalents de 1-bromo-5,8,11,-trioxadodécane sont mis à réagir dans les conditions du paragraphe 2/ de l'exemple 1 sur un équivalent de 2-7-dibromofluorène en présence de deux équivalents d'hydrure de sodium. Le monomère ainsi obtenu est suceptible d'être homopolymérise ou copolymérisé dans les conditions des paragraphes 3 et 4 de l'exemple 1.

**Exemple 3 : Préparation du monomère d'un poly-paraphénylène-vinylène possédant un bras solvatant flexible**

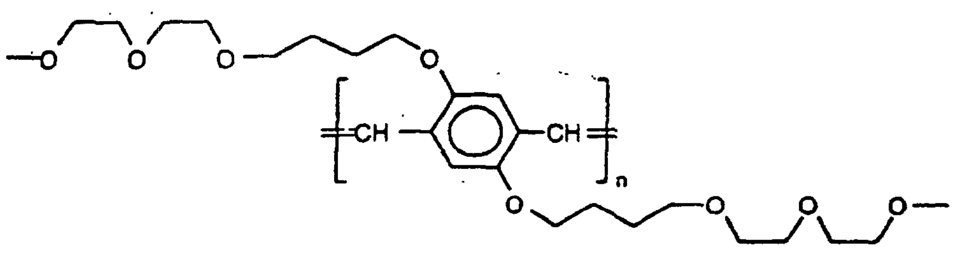

10 mmoles de 2,5 diméthyl-hydroquinone dans 25 mL de chlorobenzene, 20 mmoles de 1-b+romo-5,8,11-trioxadodécane, 20 mmoles de soude sous forme d'une solution aqueuse à 50% sont mis à réagir en présence d'un catalyseur de transfert de phase, le bromure de tetrabutyle-ammonium. Le 1,4-diméthyl-2,5-bis-(5,8,11,-trioxadodécyloxy)-benzène ainsi obtenu est purifié par chromatographie. Le 1,4-bis(bromométhyl-2,5-bis-(5,8,11,-trioxadodécyloxy)-benzène est obtenu par traitement avec deux équivalents de N-bromo-succinimide. Par élimination duplicative par le tert-butoxyde de potassium dans le THF, on obtient le polymère.

**Exemple 4 : Préparation d'une diode électroluminescente (copolymère + sel)**

On prépare d'abord une solution mère en dissolvant 50 mg de triflate de lithium ($LiCF_3SO_3$) dans 5 ml de cyclohexanone anhydre. Ensuite, 10 mg de poly[(9,9-bis(7,10,13,16-tétraoxahéptadécyle)fluorène-co-9,9-(dihéxyl) fluorène)] sont dissout dans 1 ml de cette solution mère. Le dépôt de la couche active est réalisé sur une plaque de verre (épaisseur 1 mm) recouverte d'une couche d'oxyde d'indium dopé à l'étain (épaisseur 200 nm) préalablement gravée pour délimiter des zones conductrices (cf. Figure 1). Après filtration de la solution contenant le copolymère et le sel (filtres 0,2µm), la couche active (copolymère + sel) est déposée sur le substrat verre-ITO par « spin-coating ». Le volume de solution utilisé est de 100 µl et la vitesse de rotation du plateau est de 600 tours par minute.
Dans ces conditions, on obtient un dépôt d'une épaisseur voisine de 80 nm. La dernière étape consiste à déposer à la surface de la couche active une couche métallique.
Avant tout dépôt métallique, les échantillons sont séchés sous vide primaire. L'échantillon est alors recouvert d'un masque dont les parties ajourées, qui seules laisseront passer l'aluminium, présentent la forme de l'électrode métallique. Une couche d'aluminium d'une épaisseur voisine de 120 nm est ainsi déposée par évaporation sous vide.
Lorsque le dispositif est polarisé sous une tension de 4,5 V en direct (ITO pole positif) ou en inverse (aluminium

pole positif), une émission lumineuse bleue caractéristique des polymères de type polyfluorène est observée.

**Exemple 5 : Préparation d'un diode électroluminescente (copolymère + sel + poly(oxyde d'éthylène))**

Un dispositif électroluminescent peut être préparé d'une manière similaire a celle de l'exemple 4) en partant d'une solution mère contenant 10 mg de triflate de lithium et 10 mg de poly(oxyde d'éthylène). Dans ce cas l'émission lumineuse peut-être observée pour des tensions de fonctionnement de 3,5 Volts.

## Revendications

1. Monomère de formule générale A-(Q-S)$_p$ selon laquelle

   A = noyau aromatique ou hétéroaromatique,

   $$1 \leq p \leq 6$$

   Q = radical divalent carboné ou silicié, correspondant respectivement aux formules générales :

   - $(CR^1R^2)_n$ avec $R^1$, $R^2$ = H, alkyl, alkényl comprenant entre 1 et 4 atomes de carbone et n compris entre 4 et 24, et
   - $[(O\text{-}Si(R_1R_2)]_n$ avec $R_1$,$R_2$ = H, alkyl, alkényl comprenant entre 1 et 4 atomes de carbone et n compris entre 3 et 24

   S = segment solvatant constitué d'une chaîne aliphatique comprenant au moins un hétéroatome polaire, S étant choisi dans le groupe comprenant :

   - un polyéther de formule générale :

     $$-[OCH_2CH(R)]_rOR'\text{- ou -}[OCH(R)CH_2]_rOR'$$

     avec r compris entre 1 et 50

     R = alkyle comprenant de 1 à 6 atomes de carbone
     R' = alkyle, alkényle, aryle, aryle alkyle, alkényle aryle comprenant de 1 à 30 atomes de carbone

   - une polyamine correspondant à la formule générale :

     $$-[N(R'')CH_2CH(R)]_rN(R'')R'\text{- ou -}[OCH(R)CH_2]_rN(R'')R'$$

     où :

     r compris entre 1 et 50
     R et R'' = H, alkyle comprenant de 1 à 6 atomes de carbone
     R' = alkyle, alkényle, aryle, aryl alkyle, alkényl aryle comprenant de 1 à 30 atomes de carbone

2. Monomère selon la revendication 1, **caractérisé en ce que** A est choisi dans le groupe comprenant les noyaux phényle, naphtalène, pyrrole, thiophène, benzothiophène, pyridine, quinoline, carbazole, anthracène et fluorène.

3. Monomère selon la revendication 1, **caractérisé en ce que** A est choisi dans le groupe comprenant le vinylène-phényle, vinylène-naphtalène, vinylène-pyrrole, vinylène-thiophène, vinylène-benzothiophène, vinylène-pyridine, vinylène-quinoline, carbazole, vinylène-fluorène et vinylène-anthracène.

4. Monomère selon l'une des revendications précédentes, **caractérisé en ce qu'**il présente des fonctions aptes à permettre sa polymérisation ultérieure choisies dans le groupe comprenant les fonctions halogénées, amines ou

aldéhydes.

5. Monomère selon la revendication 1 répondant à la dénomination 2,7-dibromo-9,9-bis (7,10,13,16 tétraoxaheptadécyl)fluorène.

6. Monomère selon la revendication 1, répondant à la dénomination 2,7-dibromo-9,9-bis (5,8,11-trioxadodécyl)fluorène.

7. Polymère de type homopolymère ou copolymère incorporant le monomère objet de l'une des revendications 1 à 6.

8. Dispositif organique électroluminescent constitué d'une couche mince comprenant le polymère objet de la revendication 7, ladite couche étant prise en sandwich entre deux électrodes.

9. Dispositif selon la revendication 8, **caractérisé en ce qu'**il s'agit d'une diode électroluminescente (LED).

10. Dispositif selon la revendication 8, **caractérisé en ce qu'**il s'agit d'une cellule électrochimique électroluminescente (LEC).

11. Dispositif selon la revendication 8, **caractérisé en ce que** la couche mince comprend en outre un sel dont les cations issus de la dissociation dudit sel sont choisis dans le groupe comprenant les cations dérivés des terres rares, les cations dérivés des métaux de transition, les cations dérivés des métaux alcalins, les cations dérivés des métaux alcalino-terreux, les cations ammonium ($NRi_{4+}$), phosphonium ($PRiH_{4+}$), amidinium ($RC(NRiH_2)_{2+}$) ou guanidinium ($C(NRiH_2)_{3+}$) avec R,Ri = H, alkyle, aryle comprenant 1 à 18 atomes de carbone.

12. Dispositif selon l'une des revendications 8 à 11, **caractérisé en ce que** le matériau constituant l'électrode transparente est choisi dans le groupe comprenant l'oxyde de zinc dopé par du zinc métallique, le gallium, le zirconium, l'oxyde d'étain dopé par du fluor ou de l'antimoine, l'oxyde d'indium dopé par de l'étain, l'antimoine et le fluor.

13. Dispositif selon l'une des revendication 8 à 12, **caractérisé en ce que** les électrodes sont recouvertes d'un composé choisi dans le groupe comprenant les polyquinolines, les polycarbazoles, les polymères possédant des noyaux aromatiques liés par des groupements cyanovinylènes C(CN)=CH-, les polyanylines, les polythiophènes, notamment ceux possédant des fonctions substituant éther phényle dans la position 3 et/ou éther dans la position 4, les fluorures, les oxydes, et les nitrures.

**Patentansprüche**

1. Monomer der allgemeinen Formel $A\text{-}(Q\text{-}S)_p$, gemäß welcher

A = aromatischer oder heteroaromatischer Kern,

$$1 \leq p \leq 6$$

Q = zweiwertiger Kohlenstoff- oder Siliziumrest, jeweils entsprechend den allgemeinen Formeln:

- $(CR^1R^2)_n$ mit $R^1$, $R^2$ = H, Alkyl, Alkenyl, das zwischen 1 und 4 Kohlenstoffatome enthält und wobei n zwischen 4 und 24 ist, und
- $[(O\text{-}Si(R^1, R^2)]_n$ mit $R^1$,$R^2$ = H, Alkyl, Alkenyl, das zwischen 1 und 4 Kohlenstoffatome enthält und wobei n zwischen 3 und 24 ist

S = Solvationssegment, bestehend aus einer aliphatischen Kette, die mindestens ein polares Heteroatom enthält,
wobei S aus folgender Gruppe ausgewählt ist:

- Polyether der allgemeinen Formel:

$$-[OCH_2CH(R)]_rOR'- \text{ oder } -[OCH(R)CH_2]_rOR'$$

wobei r zwischen 1 und 50 ist

R = Alkyl, das 1 bis 6 Kohlenstoffatome enthält
R' = Alkyl, Alkenyl, Aryl, Arylalkyl, Alkenylaryl, das 1 bis 30 Kohlenstoffatome enthält

- Polyamin gemäß der allgemeinen Formel:

$$-[N(R'')CH_2CH(R)]_rN(R'')R'- \text{ oder } -[OCH(R)CH_2]_rN(R'')R'$$

wobei:

r zwischen 1 und 50 ist
R und R'' = H, Alkyl, das 1 bis 6 Kohlenstoffatome enthält
R' = Alkyl, Alkenyl, Aryl, Arylalkyl, Alkenylaryl, das 1 bis 30 Kohlenstoffatome enthält.

**2.** Monomer nach Anspruch 1, **dadurch gekennzeichnet, dass** A ausgewählt ist aus der Gruppe, die die Kerne Phenyl, Naphtalin, Pyrrol, Thiophen, Benzothiophen, Pyridin, Chinolin, Carbazol, Anthracen und Fluoren enthält.

**3.** Monomer nach Anspruch 1, **dadurch gekennzeichnet, dass** A ausgewählt ist aus der Gruppe, die Vinylen-Phenyl, Vinylen-Naphtalin, Vinylen-Pyrrol, Vinylen-Thiophen, Vinylen-Benzothiophen, Vinylen-Pyridin, Vinylen-Chinolin, Carbazol, Vinylen-Fluoren und Vinylen-Anthracen enthält.

**4.** Monomer nach einem der vorhergehenden Ansprüche, **dadurch gekennzeichnet, dass** es Funktionen aufweist, die seine spätere Polymerisation ermöglichen, ausgewählt aus der Gruppe, die Halogen-, Amin- oder Aldehydfunktionen enthält.

**5.** Monomer nach Anspruch 1, das der Bezeichnung 2,7-Dibrom-9,9-bis(7,10,13,16-tetraoxaheptadodecyl)fluoren entspricht.

**6.** Monomer nach Anspruch 1, das der Bezeichnung 2,7-Dibrom-9,9-bis(5,8,11-trioxadodecyl)fluoren entspricht.

**7.** Polymer vom Homopolymer- oder Copolymer-Typ, welches das Monomer nach einem der Ansprüche 1 bis 6 enthält.

**8.** Organische Elektrolumineszenzvorrichtung, bestehend aus einer dünnen Schicht, die das Polymer nach Anspruch 7 enthält, wobei diese Schicht in Sandwichform zwischen zwei Elektroden liegt.

**9.** Vorrichtung nach Anspruch 8, **dadurch gekennzeichnet, dass** es sich um eine Elektrolumineszenzdiode (LED) handelt.

**10.** Vorrichtung nach Anspruch 8, **dadurch gekennzeichnet, dass** es sich um eine elektrolumineszente elektrochemische Zelle (LEC) handelt.

**11.** Vorrichtung nach Anspruch 8, **dadurch gekennzeichnet, dass** die dünne Schicht außerdem ein Salz enthält, dessen aus der Dissoziation dieses Salzes hervorgegangene Kationen ausgewählt sind aus der Gruppe, die Kationen, die sich von Seltenerdmetallen ableiten, Kationen, die sich von Übergangsmetallen ableiten, Kationen, die sich von Alkalimetallen ableiten, Kationen, die sich von Erdkalimetallen ableiten, Ammoniumkationen ($NRi_{4+}$), Phosphoniumkationen ($PRiH_{4+}$), Amidiniumkationen ($RC(NRiH_2)_{2+}$) oder Guanidiniumkationen ($C(NRiH_2)_{3+}$), wobei R,Ri = H, Alkyl, Aryl, das 1 bis 18 Kohlenstoffatome enthält, enthält.

**12.** Vorrichtung nach einem der Ansprüche 8 bis 11, **dadurch gekennzeichnet, dass** das Material, aus dem die transparente Elektrode besteht, ausgewählt ist aus der Gruppe, die mit metallischem Zink dotiertes Zinkoxid, Gallium, Zirconium, mit Fluor oder Antimon dotiertes Zinnoxid, mit Zinn dotiertes Indiumoxid, Antimon und Fluor enthält.

**13.** Vorrichtung nach einem der Ansprüche 8 bis 12, **dadurch gekennzeichnet, dass** die Elektroden beschichtet sind mit einer Verbindung aus der Gruppe, die Polychinoline, Polycarbazole, Polymere mit aromatischen Kernen, die durch Cyanovinylengruppierungen C(CN)=CHverbunden sind, Polyanyline, Polythiophene, insbesondere jene mit Phenylether - Substituentenfunktion in Position 3 und/oder Ether - Substituentenfunktion in Position 4, Fluoride, Oxide und Nitride enthält.

## Claims

**1.** A monomer of general formula $A\text{-}(Q\text{-}S)_p$ where:

A = aromatic or heteroaromatic nucleus,

$$1 \leq p \leq 6$$

Q = carbon-based or silicon-based divalent radical, corresponding respectively to the general formulae:

- $(CR^1R^2)_n$ where $R^1$ and $R^2$ = H, alkyl or alkenyl containing between 1 and 4 carbon atoms and n is between 4 and 24, and
- $[(O\text{-}Si(R_1R_2)]_n$ where $R_1$ and $R_2$ = H, alkyl or alkenyl containing between 1 and 4 carbon atoms and n is between 3 and 24

S = solvating segment consisting of an aliphatic chain containing at least one polar heteroatom, S being selected from the group consisting of:

- a polyether of general formula:

$$\text{-}[OCH_2CH(R)]_rOR'\text{- or -}[OCH(R)CH_2]_rOR'$$

where r is between 1 and 50

R = alkyl containing from 1 to 6 carbon atoms
R' = alkyl, alkenyl, aryl, arylalkyl or alkenylaryl containing from 1 to 30 carbon atoms

- a polyamine corresponding to the general formula:

$$\text{-}[N(R'')CH_2CH(R)]_rN(R'')R'$$

or

$$\text{-}[OCH(R)CH_2]_rN(R'')R'$$

in which:

r is between 1 and 50
R and R'' = H or alkyl containing 1 to 6 carbon atoms
R' = alkyl, alkenyl, aryl, arylalkyl or alkenylaryl containing from 1 to 30 carbon atoms.

**2.** The monomer as claimed in claim 1, **characterized in that** A is selected from the group consisting of the nuclei phenyl, naphthalene, pyrrole, thiophene, benzothiophene, pyridine, quinoline, carbazole, anthracene, and fluorene.

**3.** The monomer as claimed in claim 1, **characterized in that** A is selected from the group consisting of vinylene-phenyl, vinylene-naphthalene, vinylene-pyrrole, vinylene-thiophene, vinylene-benzothiophene, vinylene-pyridine,

vinylene-quinoline, carbazole, vinylene-fluorene, and vinylene-anthracene.

4. The monomer as claimed in one of the preceding claims, **characterized in that** it has functions allowing its subsequent polymerization which are selected from the group consisting of halogen, amine or aldehyde functions.

5. The monomer as claimed in claim 1, corresponding to the name 2,7-dibromo-9,9-bis(7,10,13,16-tetraoxaheptadecyl)fluorene.

6. The monomer as claimed in claim 1, corresponding to the name 2,7-dibromo-9,9-bis(5,8,11-trioxadodecyl)fluorene.

7. A polymer of homopolymer or copolymer type incorporating the monomer which is the subject of one of claims 1 to 6.

8. An organic electroluminescent device consisting of a thin layer comprising the polymer which is the subject of claim 7, said layer being sandwiched between two electrodes.

9. The device as claimed in claim 8, **characterized in that** it is an electroluminescent diode (LED).

10. The device as claimed in claim 8, **characterized in that** it is an electroluminescent electrochemical cell (LEC).

11. The device as claimed in claim 8, **characterized in that** the thin layer further comprises a salt whose cations resulting from the dissociation of said salt are selected from the group consisting of cations derived from rare earths, cations derived from transition metals, cations derived from alkali metals, cations derived from alkaline earth metals, the cations ammonium ($NRi_{4+}$), phosphonium ($PRiH_{4+}$), amidinium ($RC(NRiH_2)_{2+}$) or guanidinium ($C(NRiH_2)_{3+}$) where R and Ri = H, alkyl or aryl containing 1 to 18 carbon atoms.

12. A device as claimed in one of claims 8 to 11, **characterized in that** the material constituting the transparent electrode is selected from the group consisting of zinc oxide doped with metallic zinc, gallium, zirconium, tin oxide doped with fluorine or antimony, indium oxide doped with tin, antimony, and fluorine.

13. A device as claimed in one of claims 8 to 12, **characterized in that** the electrodes are covered with a compound selected from the group consisting of polyquinolines, polycarbazoles, polymers possessing aromatic nuclei linked by cyanovinylene groups C(CN)=CH-, polyanylines, polythiophenes, especially those possessing phenyl ether substituent functions in position 3 and/or ether substituent functions in position 4, fluorides, oxides, and nitrides.